# EUROPEAN PATENT APPLICATION

(11) **EP 1 959 249 A1**
(43) Date of publication of application: **20.08.2008**
(21) Application number: 06823538.1
(22) Date of filing: 01.12.2006
(51) Int. Cl.: G01N 21/35, A61B 10/00

(54) **METHOD AND APPARATUS FOR EXAMINING AND DIAGNOSING LIFE STYLE-RELATED DISEASE USING NEAR-INFRARED SPECTROSCOPY**

(30) Priority: 06.12.2005 JP 2005352158
(71) Applicant: Fatigue Science Laboratory Inc., Toyonaka-shi Osaka 560-0045 (JP); Osaka University, Suita-shi Osaka 565-0871 (JP); Kuratsune, Hirohiko, Toyonaka-shi, Osaka 560-0044 (JP)
(72) Inventor: KURATSUNE, Hirohiko, Osaka 560-0044 (JP); SAKUDO, Akikazu, Osaka 565-0871 (JP); IKUTA, Kazuyoshi, Osaka 565-0871 (JP); WATANABE, Yasuyoshi, Osaka 562-0031 (JP); NISHIZAWA, Yoshiki, Osaka 547-0043 (JP); HIRASE, Yukiyoshi, Osaka 589-0022 (JP); TAJIMA, Seiki, Osaka 543-0052 (JP); SUGANUMA, Yoshikazu, Osaka 565-0871 (JP)
(74) Representative: Behnisch, Werner
(86) International application number: PCT/JP2006/324059
(87) International publication number: WO 2007/066589

(57) **Abstract**

The present invention is a method and an apparatus for examination/diagnosis of lifestyle related disease, wherein the test sample from a human or other animal subject is irradiated with light having a wavelength region of a region of 400 nm- 2,500 nm or a part of the region, of which the reflection light, the transmission light, or the transmission reflection light is then detected to give spectroscopic data of absorbance, and afterward a previously prepared analysis model is used to execute an analysis of the absorbance at the whole or specific wavelength used for the measurement.

## Description

### Technical Field

The present invention relates to a method for examination/diagnosis of lifestyle related disease using near-infrared spectroscopy; and an apparatus used for the method.

### Background Art

Current health examination, which is generally carried out in a hospital to examine blood and the like, can not give the result immediately. Therefore, there is an increasing need for providing the results promptly along with the developments in the technique of health examination.

Health examination aims at basing on case finding (identification of disease at the earliest possible stage) to maintain and improve the health in an individual. Particularly, it aims primarily at identifying a disease at the earliest possible stage to allow appropriate health care, or taking health conditions into occupational consideration to improve health, thereby to keep fit in an individual. In addition, it also aims at grasping the health level in a group. The individual results by health examination are analyzed in relation with the group as a whole to find out influencing factors on health, allowing a measure taken for health care or labor sanitation suitable the group.

Diabetes, brain stroke, heart disease, hypertension, obesity, hyperlipemia, and diabetic nephropathy fall under the generic name of lifestyle related disease, and any one of these symptoms is identified to judge the presence of lifestyle related disease. Because any one of these symptoms appears in many cases to be succeeded by the other symptoms, it is important to identify and treat at the earliest possible stage.

Now, various fields have recently used near-infrared light to analyze components. For example, a sample is irradiated with visible light and/or near-infrared light to detect a wavelength band absorbed by a specific component, thereby to analyze quantitatively the specific component.

Concretely, the sample is put in a quartz cell, and then irradiated with visible light and/or near-infrared light having a wavelength of a region of 400 nm to 2500 nm using a near-infrared spectroscope (such as the near-infrared spectroscope NIRSystem6500 made by NIRECO corp.) to determine the reflection light, the transmission light, or the transmission reflection light.

Generally speaking, near-infrared light, which is a low energy of electromagnetic wave to have so small an absorption coefficient that it is hardly scattered by a substance, gives no damage to the sample to allow providing chemical/physical information about the sample.

Concretely, the light such as the transmission light from the irradiated sample can be detected to collect the absorbance data about the sample, which is then analyzed multivariately to provide so promptly information about the sample. For example, a biomolecule may be grasped directly and in real time to change in structure and function.

The conventional technique for such near-infrared spectrometry is described, for example, in Patent Document No.1 and No.2 below. Patent Document No.1 discloses a method for using visible and near-infrared radiation to provide information from a subject, concretely, a method to identify a group to which an unknown subject belongs, a method to identify the unknown subject, and a method to monitor the aging change of the subject in real time.

Patent Document No. 2 discloses a method for using the absorption bands of a H₂O molecule in the visible light and/or near-infrared light region to provide the absorbance data, which is then multivariately analyzed to determine the somatic cells in the milk or breast of a cow, thereby to diagnose the mastitis of the cow. As other background art, non-Patent Document No. 1 also describes clinical symptoms of lifestyle related disease and the present condition of a patient with lifestyle related disease in the Asian and Pacific district.

Non-Patent Document No. 1: Cockram CS. The epidemiology of diabetes mellitus in the Asia-Pacific region. Hong Kong Med J. 2000, 6:43-52.
Patent Document No.1: JP laid-open 2002-5827, p.1-9, Fig. 1
Patent Document No.2:WO01175420, p.1-5, Fig. 1

### Disclosure of the Invention

### [Problem to be Solved by the Invention]

As described above, examination/diagnosis of lifestyle related disease needs a simple, prompt, and high accurate method. Particularly, the examination on a large number of samples at a time have strong requirement for developing such a simple and prompt examination method.

Thus, an object of the present invention is to provide a novel method and an apparatus for using near-infrared spectrometry to examine/identify lifestyle related disease simply, promptly and highly accurately.

### [Means for Solving the Problem]

The present inventors made a strenuous study based on the above object, and have found out that near-infrared spectrometry allows examination/identification of lifestyle related disease, and that both a spectroscopic method using visible light and near-infrared light (VIS-NIR) and an analysis method of spectroscopic data thus obtained are devised to prepare an analysis model, which can be then used for good examination/identification. These findings lead to completion of the present invention.

The method and the apparatus of the present invention are characterized by a method and an apparatus for qualitative or quantitative examination/identification (diagnosis) of lifestyle related disease, wherein the test sample from a human or other animal subject is irradiated with light having a wavelength of a region of 400 nm to 2500 nm or a part of the region, of which the reflection light, the transmission light, or the transmission reflection light is then detected to give spectroscopic data of absorbance, and afterward a previously prepared analysis model is used to analyze the absorbance at a specific wavelength or over the whole wavelengths.
A quantitative model prepared by a regression analysis such as the PLS method, or a qualitative model prepared by a class discrimination analysis such as the SIMCA method is used to examine/identify lifestyle related disease. The examination/identification of lifestyle related disease as described herein means examining/identifying one or two or more terms selected from diabetes, renal dysfunction such as nephropathy, hepatic dysfunction, hypertension, hyperlipemia, obesity, heart disease, and brain stroke in reference to at least one of presence and level of the disease, as well as presence and level of the crisis risk.
In the examination/identification of plural terms of lifestyle related disease, a test sample is preferably a sample of blood (such as blood plasma or blood serum), urine, the other body fluid, tissue, tissue extract solution, or a part of living body such as ear or top ends of finger or toe. For example, collected blood or a part of living body such as finger top is used as a test sample to obtain/analyze the spectroscopic data by near-infrared spectrometry, allowing simple and prompt examination of plural terms. The obtained spectroscopic data may be used to examine simultaneously the other terms than lifestyle related disease.
As described later, in the examination/identification of the diabetes, the absorption spectroscopic data at two or more wavelengths, which are selected from a plurality of ± 5 nm wavelength regions of wavelengths selected from the group consisting of 817 nm, 921-959 nm, 987-1004 nm, 1008-1018 nm, 1028 nm, and 1040 nm, is preferably used to analyze for examination/identification of the diabetes. In the examination/identification of the diabetic renal dysfunction, the absorption spectroscopic data at two or more wavelengths, which are selected from a plurality of ± 5 nm wavelength regions of wavelengths selected from the group consisting of 835 nm, 908-912 nm, 917-963 nm, 993-1002 nm, 1008 nm-1034 nm, 1040 nm, and 1060 nm, is preferably used to analyze for examination/identification of the diabetic renal dysfunction. In the examination/identification of the other nephropathy (nephrosclerosis, glomerular nephritis, IgA nephritis, and the like) than the diabetic renal dysfunction, the absorption spectroscopic data at two or more wavelengths, which are selected from a plurality of ± 5 nm wavelength regions of wavelengths selected from the group consisting of 914-915 nm, 919-967 nm, 994 nm, 1008 nm, 1012-1014 nm, 1018 nm, 1024 nm, 1030 nm, and 1034 nm, is preferably used to analyze for examination/identification of the other nephropathy than the diabetic renal dysfunction. In the examination/identification of the hepatic dysfunction, the absorption spectroscopic data at two or more wavelengths, which are selected from a plurality of ± 5 nm wavelength regions of wavelengths selected from the group consisting of 667-679 nm, 917-955 nm, 975-1005 nm, and 1083-1085 nm, is preferably used to analyze for examination/identification of the hepatic dysfunction. Furthermore, the absorption spectroscopic data at wavelengths out of the region as described above also may be together used to analyze the examination/identification.
The analysis model of the present invention is used in the method or the apparatus of the present invention. The program for examination/diagnosis of lifestyle related disease in the present invention is used to allow a computer to prepare or update the analysis model, or execute the examination/diagnosis using the prepared analysis model.

### [Effect of the Invention]

The present invention can examine/diagnose lifestyle related disease objectively, simply, promptly, and highly accurately, and is particularly useful for examining a large number of test samples at a time because of simple and prompt examination diagnosis.
The present invention can use a test sample from blood such as blood plasma and blood serum to diagnose lifestyle related disease. In addition, urine and the other body fluid, and a part of living body such as ear and the top end of finger or toe can be non-invasively used as test samples with no damage given to a living body.
Thus, in the present invention, a test sample such as blood, blood serum or blood plasma, which is collected apart from a lesion-suspected site (local), can be analyzed to examine/diagnose various diseases associated with lifestyle related disease, while it is still unclear whether they have occurred or not. In complete physical examination of various diseases, mainly of lifestyle related disease, a blood sample is examined about known substance markers including blood sugar in diabetes, GOT or GPT in liver disease, a tumor marker in cancer, and an autoantibody in autoimmune disease. Contrarily, in the present invention, a sample is measured by near-infrared spectrometry to give the spectroscopic data, which is then analyzed to allow simple and prompt examination about a plurality of terms. In addition, the present invention, which can investigate the presence or change in a substance associated with any yet unknown disease, is very useful for screening examination of various and other diseases associated with lifestyle related disease.

### Brief Description of the Drawings

Fig. 1 is a schematic diagram illustrating a process for preparing the analysis model in the present invention and examination/diagnosis of lifestyle related disease using the prepared analysis model.
Fig. 2 is a graph showing the Coomans Plot obtained by SIMCA analysis of test samples 10 times diluted for diabetes examination in the Example of the present invention.
Fig. 3 is a graph showing discriminating power (vertical axis) at wavelength (horizontal axis) resulted from SIMCA analysis for diabetes examination in the Example of the present invention.
Fig. 4 is a graph showing the Coomans Plot obtained by SIMCA analysis of test samples 10 times diluted for diabetic nephropathy examination in the Example of the present invention.
Fig. 5 is a graph showing discriminating power (vertical axis) at wavelength (horizontal axis) resulted from SIMCA analysis for diabetic nephropathy examination in the Example of the present invention.
Fig. 6 is a graph showing the Coomans Plot obtained by SIMCA analysis of test samples 10 times diluted for examination on patients with the other nephropathy than diabetic renal dysfunction in the Example of the present invention.
Fig. 7 is a graph showing discriminating power (vertical axis) at wavelength (horizontal axis) resulted from SIMCA analysis for examination on patients with the other nephropathy than diabetic renal dysfunction in the Example of the present invention.
Fig. 8 is a graph showing the Coomans Plot obtained by SIMCA analysis of test samples 10 times diluted for examination on patients with hepatic dysfunction in the Example of the present invention.
Fig. 9 is a graph showing discriminating power (vertical axis) at wavelength (horizontal axis) resulted from SIMCA analysis for examination on patients with hepatic dysfunction in the Example of the present invention.

### Description of the Preferred Embodiment

As one embodiment of the present invention, an apparatus for examining/diagnosing lifestyle related disease (hereinafter referred to as "the present apparatus") will be described in reference to drawings below.

### [1] VIS-NIR spectrometry by the present apparatus and data analysis method

### [1.1] Outline of VIS-NIR spectrometry

The present apparatus uses the method of the present invention to examine/diagnose lifestyle related disease, wherein (a) the test sample from a human or other animal subject is irradiated with light having a wavelength of a region of 400 nm to 2500 nm or a part of the region, (b) the reflection light, the transmission light, or the transmission reflection light is then detected to give spectroscopic data of absorbance, and afterward (c) a previously prepared analysis model is used to analyze the absorbance at a specific wavelength or over the whole wavelengths

The present apparatus is primarily characterized by allowing objective, simple, prompt, and highly accurate diagnosis of lifestyle related disease. The test sample is irradiated with light having a wavelength of a region of 400 nm-2500 nm or a part of the region (such as 600-1100 nm). After preparing the model, this region of wavelength is set to divide into one or a plurality of fractional regions of wavelength which contain the lights having wavelengths necessary for using the prepared analysis model to examine/identify.

The light source to use includes, but is not limited to, a halogen lamp and a LED. Light emitted from the light source is irradiated on a test sample directly or through an illumination means such as a fiber probe. As described later, a pre-spectroscopic way may be employed to work a spectroscope before irradiating the test sample, or a post-spectroscopic way may be employed to work after. The pre- spectroscopic way is carried out by one method of using a prism to spectroscope the light from a light source at a time, or by another method of changing the slit width of a diffraction grating to change wavelength consecutively. The latter method resolves the light from a light source into certain wavelength widths to irradiate a test sample with light which is consecutively varied in wavelength. In the Example as described later, the light within the region of 600-1100 nm is resolved by 2 nm of wavelength resolution, and the light consecutively varied in wavelength by every resolution of 2 nm was irradiated the test sample.

The reflection light, the transmission light, or the transmission reflection light of the light irradiated on the test sample is detected by a detector to provide a raw spectroscopic data of absorbance. The raw spectroscopic data of absorbance may directly be used for examination/identification by an analysis model. The data is preferably treated to convert, for example, by using a spectroscopic procedure or a multivariate procedure to resolve peaks in the provided spectrum into the elemental peaks, and the converted data is then used for examination/identification by the analysis model. The spectroscopic procedure includes secondary differentiation or Fourier transform, and the multivariate procedure includes Weblet transform or neural network method, but they are not particularly limited.

### [1.2] Data analysis method (preparation of analysis model)

The present apparatus uses an analysis model to analyze the absorbance at a certain wavelength (or over whole measure wavelengths) in the spectroscopic data of absorbance as provided above, thereby to examine/diagnose lifestyle related disease. Thus, the analysis model must be previously prepared for examination/diagnosis at the final step. The analysis model may be simultaneously prepared with the spectroscopic measurement.

Preferably, the analysis model is previously prepared before the measurement. Alternatively, the spectroscopic data obtained by the measurement may be divided into one data for preparing the analysis model and another data for examining/diagnosing, and the former data is used to prepare the analysis model, which is then used for examination/diagnosis. For example, when a large number of test samples are examined at a time, a part of them is used to prepare the analysis model. In other words, the analysis model is simultaneously prepared with the spectroscopic measurement. The procedure can prepare the analysis model without teacher's data, allowing coping with both the quantitative model and the qualitative model.

The analysis model can be prepared by multivariate analysis. For example, diabetes, nephropathy, or hepatic dysfunction is anticipated for examination of lifestyle related disease by decomposing with singular-value decomposition a data matrix storing the absorption spectrum obtained over the whole wavelengths into scores and loadings to extract principal components estimating differences in presence or degree of a disease (progression or seriousness of the disease) in the test sample (principal component analysis). This allows multiple regression analysis to use independent components which are low in collinearity (=high correlation among explanatory variables). The multiple regression analysis can be applied by allocating score to the explanatory variables and the response variable to the presence or degree of the disease. This can prepare an analysis model which bases on the absorption spectrum over the whole measurement wavelengths or at a specific wavelength to estimate the presence or degree of the disease. These serial procedures (multivariate analysis) are established as Principal Component Regression (PCR) or PLS (Partial Least Squares) regression (see: Yukihiro Ozaki, Akihumi Uda, Toshio Akai, "Multivariate Analysis for Chemist-Introduction to Chemometrix", Kodansha Co., Ltd., 2002). Regression analysis includes additionally CLS (Classical Least Squares) and Cross Variation.

The method as described above prepares a quantitative analysis model. The qualitative analysis model can be prepared by applying multivariate analysis such as Principal Component Analysis (PCA) for class analogy, SIMCA (Soft Independent Modeling of Class Analogy), and KNN (K Nearest Neighbors). The SIMCA analyzes a plurality of groups (classes) by principal component analysis to prepare their respective principal component models. An unknown test sample is compared with every principal component model to assign the sample to a class of the most fitting model. The class analogy analysis such as SIMCA can be a method wherein an absorption spectrum or a regression spectrum is recognized by pattern to be classified into each class.

The analysis model using the multivariate analysis such as the SIMCA or the PLS described above can be prepared by employing a self-made software or a commercially available multivariate analysis software. A software specified as a program for examining/diagnosing lifestyle related disease may be prepared to allow prompt analysis.

Such multivariate analysis software is used to prepare an analysis model as a file to store, which is then called up to examine/diagnose about an unknown test sample, allowing quantitative or qualitative examination/diagnosis using the analysis model. Thus, this allows simple and prompt examination/diagnosis of lifestyle related disease. A plurality of analysis models including a quantitative model and a qualitative model are stored as files, which are preferably updated into appropriate ones.

Thus, the examination/diagnosis program (analysis software) of the present invention allows a computer to execute preparation, updating of the analysis model or examination/diagnosis of lifestyle related disease by using the prepared analysis model with spectroscopic data of a sample. The program of the present invention can be provided as a recording medium in which the program is stored as readable by the computer. Such a storage medium includes, but is not limited to, a magnetic storage medium such as a flexible disk, a hard disk, and a magnetic tape; an optical storage medium such as CD-ROM, CD-R, CD-RW, DVD-ROM, DVD-RAM, and DVD-RW; an electric storage medium such as RAM and ROM; and a magnetic/optical storage medium such as MO.

The analysis model prepared can determine what wavelength of light is necessary for examination/diagnosis using the analysis model. The present apparatus can be simplified in construction by designing to irradiate a test sample with a single or a plurality of wavelength regions of lights so determined.
Since the study revealed that 817 nm, 921-959 nm, 987-1004 nm, 1008-1018 nm, 1028 nm, and 1040 nm were effective wavelengths for examination/diagnosis of diabetes, it is preferable that diabetes is examined by analysis using the absorbance spectroscopic data at two or more (preferably 2-15 or about 5-10) wavelengths selected from a plurality of wavelength regions consisting of these wavelength's respective ± 5 nm scopes. Similarly, since it was revealed that 835 nm, 908-912 nm, 917-963 nm, 993-1002 nm, 1008-1034 nm, 1040 nm, and 1060 nm were effective wavelengths for examination/diagnosis of diabetic renal dysfunction, it is preferable that diabetic renal dysfunction is examined by analysis using the absorbance spectroscopic data at two or more (preferably 2-15 or about 5-10) wavelengths selected from a plurality of wavelength regions consisting of these wavelength's respective ± 5 nm scopes. Further, since it was revealed that 914-915 nm, 919-967 nm, 994 nm, 1008 nm, 1012-1014 nm, 1018 nm, 1024 nm, 1030 nm, and 1034 nm were effective wavelengths for examination/diagnosis of the other nephropathy (nephrosclerosis, glomerular nephritis, IgA nephritis, and the like) than diabetic renal dysfunction, it is preferable that the other nephropathy than diabetic renal dysfunction is examined by analysis using the absorbance spectroscopic data at two or more (preferably 2-15 or about 5-10) wavelengths selected from a plurality of wavelength regions consisting of these wavelength's respective ± 5 nm scopes. Furthermore, since it was revealed that 667-679 nm, 917-955 nm, 975-1005 nm, and 1083-1085 nm were effective wavelengths for examination/diagnosis of hepatic dysfunction, it is preferable that hepatic dysfunction is examined by analysis using the absorbance spectroscopic data at two or more (preferably 2-15 or about 5-10) wavelengths selected from a plurality of wavelength regions consisting of these wavelength's respective ± 5 nm scopes. In these examinations, the analysis model is used to analyze the absorbance spectroscopic data at a plurality of the selected wavelengths and the added absorbance spectroscopic data at the other wavelengths.

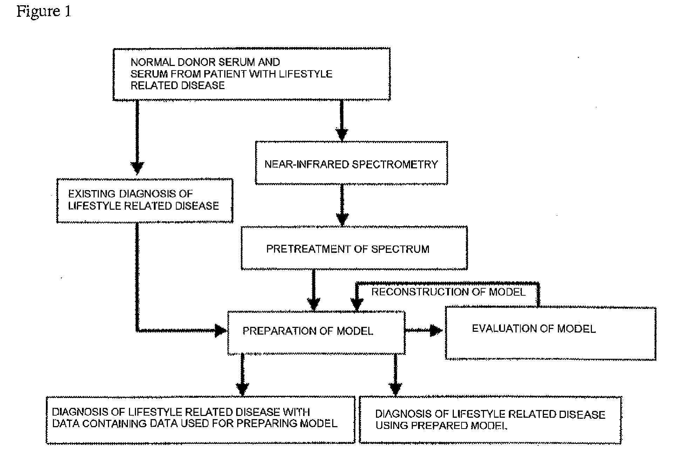

The step for preparing an analysis model and the step for using the model to examine/diagnose lifestyle related disease as described above are summarized schematically in Fig. 1. In the step for preparing an analysis model, for example, blood serum samples from a normal person and a patient with lifestyle related disease are measured by near-infrared spectrometry to give spectroscopic data, which is then pre-treated and subjected to multivariate analysis in reference with the results of existing examination/diagnosis of lifestyle related disease to prepare the analysis model. The analysis model thus prepared is used to examine/diagnose an unknown sample about lifestyle related disease, if appropriate, together with the data (wavelength information) used for preparing the analysis model, allowing evaluation of the analysis model. An analysis model, which is evaluated to have higher performance than a formerly prepared one, may be employed to update and then the model is reconstructed appropriately.
In the Examples as described later, a 10x diluted blood serum was used as a test sample, and irradiated consecutively thrice to give three respective absorbance data, which were then used to prepare the analysis model. An analysis model can be prepared in this way, and an unknown sample can be measured by spectrometry in the similar way to give absorbance data, which is then analyzed using the analysis model to allow examination/diagnosis of lifestyle related disease.

### [2] Specific construction of the present apparatus

The examination/diagnosis system of the present apparatus comprises four elements: (i) a probe (irradiator), (ii) a spectroscope/detector, (iii) a data analyzer, and (iv) a result display. The elements will be each described below.

### [2.1] Probe (Irradiator)

The probe has a function introducing light (having a wavelength of a region of 400 nm-2500 nm or a part thereof) from a light source such as a halogen lamp and LED into a test sample to determine. There is mentioned as the fiber probe a system for irradiating an object (test sample) to determine with light through a flexible optical fiber. Generally, the probe for a near-infrared spectroscope can be inexpensively prepared and is available by a low cost.

The system may be designed to irradiate directly an object (test sample) to determine with light emitted from a light source. The case needs no probe, and the light source serves as a light irradiator.

As described above, the analysis model prepared can determine what wavelength of light is necessary for examination/diagnosis using the analysis model. The present apparatus can be simplified in construction by designing to irradiate a test sample with a single or a plurality of wavelength regions of lights so determined.

### [2.2] Spectroscope/detector (spectroscoping means and detecting means)

The measurement system of the present apparatus has a near-infrared spectroscope. The near-infrared spectroscope generally irradiates an object to determine with light, of which the reflection light, the transmission light or the transmission reflection light from the object is detected by a detector. Further, it determines the wavelength-depending absorbance of the detected light to the incident light.

Spectroscopy is divided in way into pre-spectroscopy and post-spectroscopy. The former spectroscopes light before an object to determine is irradiated. The latter detects light from the object to spectroscope the light. The spectroscope/detector of the present apparatus may take any way of pre-spectroscopy and post-spectroscopy.

There are three kinds of detection methods: reflection light detection method, transmission light detection method, and transmission reflection light detection method. The reflection light detection method and the transmission light detection method uses a detector to detect the reflection light and the transmission light. The transmission reflection light detection method detects the light which incident light refracts and reflects inside the object to emit again outside the object. The spectroscope/detector in the present apparatus may adopt the reflection light detection method, the transmission light detection method or the transmission reflection light detection method.

The detector in the spectroscope/detector, for example, may comprise, but is not limited to, a CCD (Charge Coupled Device) which is a semiconductor device, and may use the other light receiving device. The spectroscope may comprise a known means.

### [2.3] Data analyzer (data analyzing means)

The wavelength-depending absorbance which is an absorbance spectroscopic data is provided by the spectroscope/detector. The data analyzer bases on the absorbance spectroscopic data to use the analysis model previously prepared as described above, thereby to diagnose lifestyle related disease.

A plurality of analysis models including a quantitative model and a qualitative model is preferably prepared. They may be appropriately used depending on whether the data is quantitatively or qualitatively evaluated.

The data analyzer may comprise a storage part for storing various data including spectroscopic data, a multivariate analysis program and an analysis model, and an operation part for basing on these data and the program to operate. The storage and operation can be achieved, for example, by an IC chip. The present apparatus can be easily small-sized to be a handheld one. The analysis model as described above is also written in the storage part such as the IC chip.

### [2.4] Result display (displaying means)

The result display shows an analysis result obtained in the data analyzer. Specifically, it displays the presence or the risk of lifestyle related disease given by analysis using an analysis model. For example, basing on the result of class judgment given by analysis using the qualitative model, it displays "diabetes", "highly possible diabetes", "lowly possible diabetes", or "normal person". The result display is preferably a flat display made by liquid crystal in order to prepare a handheld apparatus.

As described above, the present apparatus can be used for examination/diagnosis of lifestyle related disease. The examination/diagnosis of lifestyle related disease means not only to examine/diagnose whether a subject suffers from a specific lifestyle related disease or not, but also to evaluate quantitatively the progression or the seriousness of the disease and to judge the risk by class, thereby to examine/diagnose various aspects of the disease.

### Example

The present invention will be described in reference to Examples below to demonstrate that near-infrared spectrometry allows examination/diagnosis of lifestyle related disease, but is not limited by the Examples.

### [1.1] Measurement of absorption spectrum

The present Example used a following measurement method to measure the absorption spectrum of each sample.
A normal donor serum, and sera from patients with diabetes, diabetic renal dysfunction, the other nephropathy than diabetic renal dysfunction, and hepatic dysfunction were each 10x diluted with PBS buffer to use as test samples.

1 mL of each test sample was put in a polystyrene cuvette, and measured by the near-infrared spectroscopic apparatus (FQA-NIRGUN[Japan Fantec Research Institute, Shizuoka, Japan]). Specifically, each test sample was irradiated consecutively thrice with light having a wavelength of 600-1100 nm to detect their respective reflection lights, thereby to determine absorption spectra. The wavelength resolution was 2 nm. The light path across the test sample was set to have a length of 10 mm.

### [1.2.1] Analysis of absorption spectrum (diabetes)

In the present Example, the absorption spectrum thus obtained was subjected to multivariate analysis by the SIMCA method to prepare the analysis model for diabetes. The diabetes was diagnosed according to the existing diagnosis standard by Ministry of Health and Welfare of Japan. The classification by the SIMCA method defined a patient with diabetes as Class 4 and a normal person as Class 1.

In the present Example, in order to prepare the analysis model, the commercially available multivariate analysis software (trade name: Pirouette ver.3.01 [Informetrics]) was used to execute the SIMCA analysis using algorithms shown in Table 1 below. 78 samples of normal donor sera and 22 samples of sera from patients with diabetes, totally 100 samples were used to prepare the model by the SIMCA analysis. The SIMCA-analyzed model was investigated to be useful for diagnosis of lifestyle related disease depending on whether it could rightly diagnose or not 11 samples of normal donor sera and 12 samples of sera from patients with diabetes all of which had been excluded from preparing the model.

**[Table 1]**

| SIMCA | |
|---|---|
| # of Included Samples: | 300 |
| # of Included X vars: | 253 |
| Class Variable: | Class |
| Preprocessing: | Mean-center |
| Scope: | Local |
| Maximum factors: | 30 |
| Optimal factors: | 30,9 |
| Prob threshold: | 0.9500 |
| Calib Transfer: | Not enabled |
| Transforms: | |
| None | |

Briefly explaining the algorithms as described above, "# of Included Samples" is a sample number used for the analysis, and a sample number of 300 means that 100 samples were irradiated consecutively thrice to give three respective absorbance data, which were then used.

"Preprocessing" means a pre-treatment, and "Mean-center" shows that an original point for plotting is shifted to the center of a data set. "Scope" includes a Global one and a Local one, and the Local one was selected. "Maximum factors" is a Factor (principal component) number to analyze at the maximum, and up to 30 was selected. "Optimal Factors" is an optimal Factor number for preparing a model which is found out from analysis result, and "30, 9" shows that up to Factor 30 is optimal for Class 1 and up to Factor 9 is optimal for Class 4. "Probability threshold" is a threshold value used to determine whether a subject belongs to a certain class or not. "Calibration transfer" shows whether mathematical adjustment is required to alleviate the difference between apparatuses or not. "Transform" shows a transformation, and "None" shows no transformation treatment.

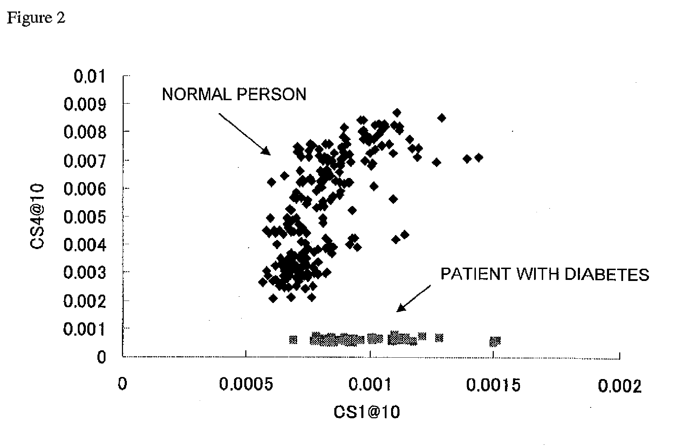

Fig. 2 shows a Coomans Plot obtained by the SIMCA analysis in the present Example. Table 2 as shown below shows a result of Interclass Distances, and Table 3 shows a result of Misclassification.

**[Table 2]**

| | CS1@10 | CS4@10 |
|---|---|---|
| CS1 | 0 | 4.316204 |
| CS4 | 4.316204 | 0 |

In Table 2, CS1 and CS2 show Class 1 and Class 2, respectively (hereinafter same). "CS1@10" means that Class 1 uses 10 Factors (principal components), and hereinafter similarly, the number value following @ shows a number of used Factors.

**[Table 3]**

| | Pred1@10 | Pred4@10 | No match |
|---|---|---|---|
| Actual 1 | 234 | 0 | 0 |
| Actual4 | 0 | 66 | 0 |

In Table 3, "Actual1" means that the actual class is "1", and hereinafter similarly. "Pred1" means that the class, which is anticipated using the analysis model prepared by SIMCA analysis in the present Example, is "1", and hereinafter similarly. "No match" is a numerical value of cases which are judged to be neither patients with diabetes nor normal persons. These results reveal that the analysis model prepared by SIMCA analysis can be used to judge rightly whether the subject is a patient with diabetes or not.

Then, it was investigated whether the analysis model prepared by SIMCA analysis could be used to diagnose an unknown test sample which had been excluded from preparing the model or not. The results are shown in Table 4 below.

**[Table 4]**

| Excluded samples | | | |
|---|---|---|---|
| | PredCS1@10 | PredCS4@10 | No match |
| ActualCS1 | 33 | 0 | 0 |
| ActualCS4 | 3 | 14 | 3 |
| Unmodeled | 0 | 0 | 0 |

In Table 4, "ActualCS1" means that the actual class is "1", and hereinafter similarly. "PredCS1" means that the class, which is anticipated using the analysis model prepared by the SIMCA analysis, is "1", and hereinafter similarly. "No match" is a numerical value of the case which is judged to be neither a patient with diabetes nor a normal person. "Excluded samples" is a result of unknown samples to anticipate. Table 4 shows that the unknown samples can be diagnosed about diabetes.

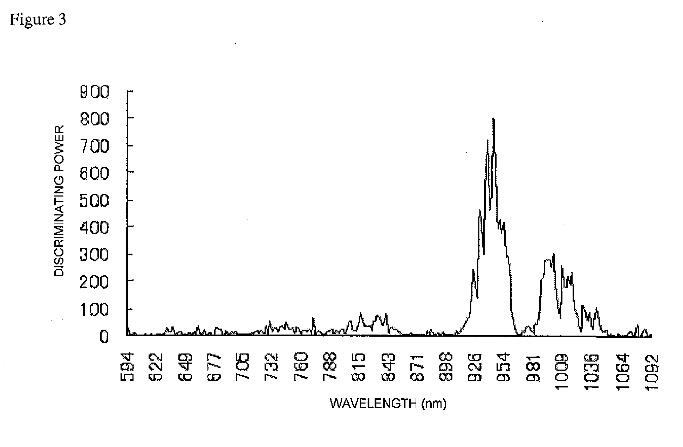

Fig. 3 shows the discriminating power (vertical axis) at wavelength (horizontal axis) which was obtained from the result by the SIMCA analysis. The figure shows that the higher discriminating power at a wavelength, the more different the wavelength between two classes. Thus, the wavelength, which corresponds to a sharp peak having a high discriminating power, is thought to be effective for discriminating serum between a normal person and a patient with diabetes. Therefore, the identification with the wavelength taught by the SIMCA analysis allows simple, prompt, and accurate diagnosis of diabetes.

The analysis model prepared in this way is stored as a file, which is called out for examining/diagnosing an unknown test sample to anticipate in which class it is classified. This allows simple and prompt examination/diagnosis of diabetes.

### [1.2.2] Analysis of absorption spectrum (diabetic nephropathy)

In the present Example, the absorption spectrum thus obtained was subjected to multivariate analysis by the SIMCA method to prepare the analysis model for nephropathy. The nephropathy was diagnosed according to the existing diagnosis standard by Ministry of Health and Welfare of Japan. The classification by the SIMCA method defined a patient with nephropathy as Class 5 and a normal person as Class 1.

In the present Example, in order to prepare the analysis model, the commercially available multivariate analysis software (trade name: Pirouette ver.3.01 [Informetrics]) was used to execute the SIMCA analysis using algorithms shown in Table 5 below. 78 samples of normal donor sera and 23 samples of sera from patients with nephropathy, totally 101 samples were used to prepare the model by the SIMCA analysis. The SIMCA-analyzed model was investigated to be useful for diagnosis of lifestyle related disease depending on whether it could rightly diagnose or not 11 samples of normal donor sera and 7 samples of sera from patients with nephropathy all of which had been excluded from preparing the model.

**[Table 5]**

| SIMCA | |
|---|---|
| # of Included Samples: | 303 |
| # of Included X vars: | 253 |
| Class Variable: | Class |
| Preprocessing: | Mean-center |
| Scope: | Local |
| Maximum factors: | 30 |
| Optimal factors: | 30,12 |
| Prob threshold: | 0.8500 |
| Calib Transfer: | Not enabled |
| Transforms: None | |

Briefly explaining the algorithms as described above, "# of Included Samples" is a sample number used for the analysis, and a sample number of 303 means that 101 samples were irradiated consecutively thrice to give three respective absorbance data, which were then used.

"Preprocessing" means a pre-treatment, and "Mean-center" shows that an original point for plotting is shifted to the center of a data set. "Scope" includes a Global one and a Local one, and the Local one was selected. "Maximum factors" is a Factor (principal component) number to analyze at the maximum, and up to 30 was selected. "Optimal Factors" is an optimal Factor number for preparing a model which is found out from analysis result, and "30, 12" shows that up to Factor 30 is optimal for Class 1 and up to Factor 12 is optimal for Class 5. "Probability threshold" is a threshold value used to determine whether a subject belongs to a certain class or not. "Calibration transfer" shows whether mathematical adjustment is required to alleviate the difference between apparatuses or not. "Transform" shows a transformation, and "None" shows no transformation treatment.

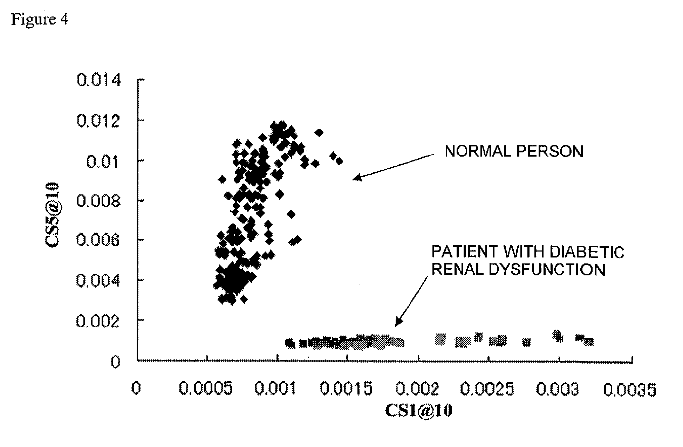

Fig. 4 shows a Coomans Plot obtained by the SIMCA analysis in the present Example. Table 6 as shown below shows a result of Interclass Distances, and Table 7 shows a result of Misclassification.

**[Table 6]**

| | CS1@10 | CS5@10 |
|---|---|---|
| CS1 | 0 | 4.876747 |
| CS5 | 4.876747 | 0 |

In Table 6, CS1 and CS5 show Class 1 and Class 5, respectively. "CS1@10" means that Class 1 uses 10 Factors (principal components).

**[Table 7]**

| | Pred1@10 | Prect5@10 | No match |
|---|---|---|---|
| Actual1 | 234 | 0 | 0 |
| Actual5 | 0 | 69 | 0 |

In Table 7, "Actual1" means that the actual class is "1", and hereinafter similarly. "Pred1" means that the class, which is anticipated using the analysis model prepared by SIMCA analysis in the present Example, is "1", and hereinafter similarly. "No match" is a numerical value of the case which is judged to be neither a patient with diabetic nephropathy nor a normal person. These results reveal that the analysis model prepared by SIMCA analysis can be used to judge rightly whether the subject is a patient with nephropathy or not.

Then, it was investigated whether the analysis model prepared by SIMCA analysis could be used to diagnose an unknown test sample which had been excluded from preparing the model or not. The results are shown in Table 8 below.

**[Table 8]**

| Excluded samples | | | |
|---|---|---|---|
| | PredCS1@10 | PredCS5@10 | No match |
| ActualCS1 | 33 | 0 | 0 |
| ActualCS5 | 0 | 21 | 0 |

In Table 8, "ActualCS1" means that the actual class is "1", and hereinafter similarly. "PredCS1" means that the class, which is anticipated using the analysis model prepared by the SIMCA analysis, is "1", and hereinafter similarly. "No match" is a numerical value of the case which is judged to be neither a patient with diabetic nephropathy nor a normal person. "Excluded samples" is a result of unknown samples to anticipate. Table 8 shows that the unknown samples can be diagnosed about nephropathy.

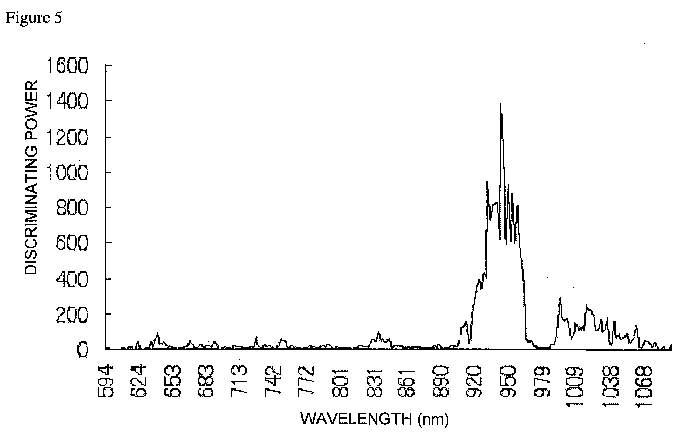

Fig. 5 shows the discriminating power (vertical axis) at wavelength (horizontal axis) which was obtained from the result by the SIMCA analysis. The figure shows that the higher discriminating power at a wavelength, the more different the wavelength between two classes. Thus, the wavelength, which corresponds to a sharp peak having a high discriminating power, is thought to be effective for discriminating serum between a normal person and a patient with diabetic nephropathy. Therefore, the identification with the wavelength taught by the SIMCA analysis allows simple, prompt, and accurate diagnosis of diabetic nephropathy.

The analysis model prepared in this way is stored as a file, which is called out for examining/diagnosing an unknown test sample to anticipate in which class it is classified. This allows simple and prompt examination/diagnosis of diabetic nephropathy.

### [1.2.3] Analysis of absorption spectrum (patient with the other nephropathy than diabetic renal dysfunction)

In the present Example, the absorption spectrum thus obtained was subjected to multivariate analysis by the SIMCA method to prepare the analysis model for patient with the other nephropathy than diabetic renal dysfunction. The classification by the SIMCA method defined a patient with the other nephropathy than diabetic renal dysfunction as Class 6 and a normal person as Class 1.

In the present Example, in order to prepare the analysis model, the commercially available multivariate analysis software (trade name: Pirouette ver.3.01 [Informetrics]) was used to execute the SIMCA analysis using algorithms shown in Table 9 below. 79 samples of normal donor sera and 23 samples of sera from patients with the other nephropathy than diabetic renal dysfunction, totally 102 samples were used to prepare the model by the SIMCA analysis. The SIMCA-analyzed model was investigated to be useful for diagnosis of lifestyle related disease depending on whether it could rightly diagnose or not 10 samples of normal donor sera and 7 samples of sera from patients with the other nephropathy than diabetic renal dysfunction all of which had been excluded from preparing the model.

**[Table 9]**

| SIMCA | |
|---|---|
| # of Included Samples: | 306 |
| # of Included X vars: | 253 |
| Class Variable: | Class |
| Preprocessing: | Mean-center |
| Scope: | Local |
| Maximum factors: | 30 |
| Optimal factors: | 30,10 |
| Prob threshold: | 0.9500 |
| Calib Transfer: | Not enabled |
| Transforms: | |
| None | |

Briefly explaining the algorithms as described above, "# of Included Samples" is a sample number used for the analysis, and a sample number of 306 means that 103 samples were irradiated consecutively thrice to give three respective absorbance data, which were then used.

"Preprocessing" means a pre-treatment, and "Mean-center" shows that an original point for plotting is shifted to the center of a data set. "Scope" includes a Global one and a Local one, and the Local one was selected. "Maximum factors" is a Factor (principal component) number to analyze at the maximum, and up to 30 was selected. "Optimal Factors" is an optimal Factor number for preparing a model which is found out from analysis result, and "30, 10" shows that up to Factor 30 is optimal for Class 1 and up to Factor 10 is optimal for Class 6. "Probability threshold" is a threshold value used to determine whether a subject belongs to a certain class or not. "Calibration transfer" shows whether mathematical adjustment is required to alleviate the difference between apparatuses or not. "Transform" shows a transformation, and "None" shows no transformation treatment.

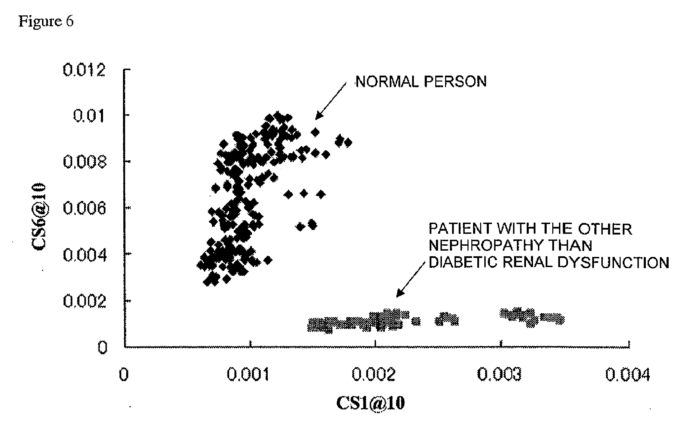

Fig. 6 shows a Coomans Plot obtained by the SIMCA analysis in the present Example. Table 10 as shown below shows a result of Interclass Distances, and Table 11 shows a result of Misclassification.

**[Table 10]**

| | CS1@5 | CS6@5 |
|---|---|---|
| CS1 | 0 | 3.615742 |
| CS6 | 3.615742 | 0 |

In Table 10, CS1 and CS6 show Class 1 and Class 6, respectively. "CS1@5" means that Class 1 uses 5 Factors (principal components).

**[Table 11]**

| | Pred1@5 | Pred6@5 | No match |
|---|---|---|---|
| Actual1 | 237 | 0 | 0 |
| Actual6 | 0 | 69 | 0 |

In Table 11, "Actual1" means that the actual class is "1", and hereinafter similarly. "Pred1" means that the class, which is anticipated using the analysis model prepared by SIMCA analysis in the present Example, is "1", and hereinafter similarly. "No match" is a numerical value of the case which is judged to be neither a patient with the other nephropathy than diabetic renal dysfunction nor a normal person. These results reveal that the analysis model prepared by SIMCA analysis can be used to judge rightly whether the subject is a patient with nephropathy or not.

Then, it was investigated whether the analysis model prepared by SIMCA analysis could be used to diagnose an unknown test sample which had been excluded from preparing the model or not. The results are shown in Table 12 below.

**[Table 12]**

| Excluded samples | | | |
|---|---|---|---|
| | PredCS1@5 | PredCS6@5 | No match |
| ActualCS1 | 30 | 0 | 0 |
| ActualCS5 | 3 | 18 | 0 |

In Table 12, "ActuaICS1" means that the actual class is "1", and hereinafter similarly. "PredCS1" means that the class, which is anticipated using the analysis model prepared by the SIMCA analysis, is "1", and hereinafter similarly. "No match" is a numerical value of the case which is judged to be neither a patient with the other nephropathy than diabetic renal dysfunction nor a normal person. "Excluded samples" is a result of unknown samples to anticipate. Table 12 shows that the unknown samples can be diagnosed about a patient with the other nephropathy than diabetic renal dysfunction.

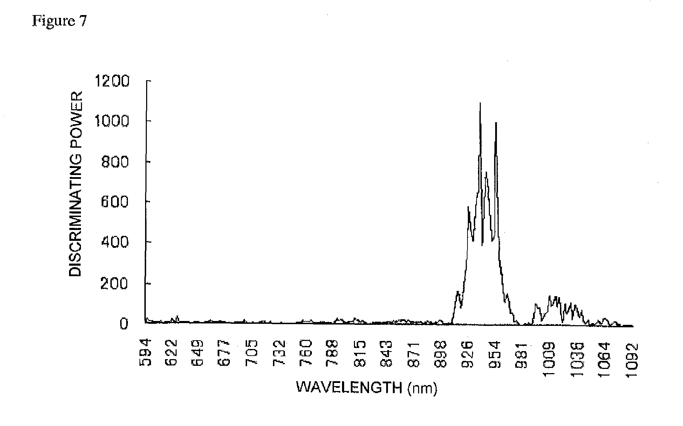

Fig. 7 shows the discriminating power (vertical axis) at wavelength (horizontal axis) which was obtained from the result by the SIMCA analysis. The figure shows that the higher discriminating power at a wavelength, the more different the wavelength between two classes. Thus, the wavelength, which corresponds to a sharp peak having a high discriminating power, is thought to be effective for discriminating serum between a normal person and a patient with the other nephropathy than diabetic renal dysfunction. Therefore, the identification with the wavelength taught by the SIMCA analysis allows simple, prompt, and accurate diagnosis of the other nephropathy than diabetic renal dysfunction.

The analysis model prepared in this way is stored as a file, which is called out for examining/diagnosing an unknown test sample to anticipate in which class it is classified. This allows simple and prompt examination/diagnosis of a patient with the other nephropathy than diabetic renal dysfunction.

### [1.2.4] Analysis of absorption spectrum (hepatic dysfunction)

In the present Example, the absorption spectrum thus obtained was subjected to multivariate analysis by the SIMCA method to prepare the analysis model for patient with hepatic dysfunction. The hepatic dysfunction was diagnosed according to the existing diagnosis standard by Ministry of Health and Welfare of Japan, including abnormality in numerical value of GOT (glutamic-oxaloacetic transaminase), GPT (glutamic-pyruvic transaminase), and γ-GTP (γ-glutamyl transpeptidase). The classification by the SIMCA method defined a patient with hepatic dysfunction as Class 2 and a normal person as Class 1.

In the present Example, in order to prepare the analysis model, the commercially available multivariate analysis software (trade name: Pirouette ver.3.01 [Informetrics]) was used to execute the SIMCA analysis using algorithms shown in Table 13 below. 74 samples of normal donor sera and 22 samples of sera from patients with hepatic dysfunction, totally 96 samples were used to prepare the model by the SIMCA analysis. The SIMCA-analyzed model was investigated to be useful for diagnosis of lifestyle related disease depending on whether it could rightly diagnose or not 15 samples of normal donor sera and 15 samples of sera from patients with hepatic dysfunction all of which had been excluded from preparing the model.

**[Table 13]**

| SIMC A | |
|---|---|
| # of Included Samples: | 288 |
| # of Included X vars: | 253 |
| Class Variable: | Class |
| Preprocessing: | Mean-center |
| Scope: | Local |
| Maximum factors: | 20 |
| Optimal factors: | 20, 20 |
| Prob threshold: | 0.9500 |
| Calib Transfer: | Not enabled |
| Transforms: | |
| Smooth(25) | |
| SNV | |

Briefly explaining the algorithms as described above, "# of Included Samples" is a sample number (spectrum number) used for the analysis, and a sample number of 288 means that 96 samples were irradiated consecutively thrice to give three respective absorbance data, which were then used.

"Preprocessing" means a pre-treatment, and "Mean-center" shows that an original point for plotting is shifted to the center of a data set. "Scope" includes a Global one and a Local one, and the Local one was selected. "Maximum factors" is a Factor (principal component) number to analyze at the maximum, and up to 30 was selected. "Optimal Factors" is an optimal Factor number for preparing a model which is found out from analysis result, and "20, 20" shows that up to Factor 20 is optimal for Class 1 and up to Factor 20 is optimal for Class 2. "Probability threshold" is a threshold value used to determine whether a subject belongs to a certain class or not. "Calibration transfer" shows whether mathematical adjustment is required to alleviate the difference between apparatuses or not. "Transform" shows a transformation, and "Smooth" shows smoothing. Basing on the principle of multinominal filter by Savitzky-Golay, smoothing transformation was carried out by regression to the explanatory variables within the windows comprising the central data point and n one-side points where n=25 was selected. The SNV is a method to correct dispersion. Firstly, sample variables are treated to calculate a standard deviation and a mean. Then, the dispersion is corrected by subtracting the mean from each variable value to give a value, which is then divided by the standard deviation.

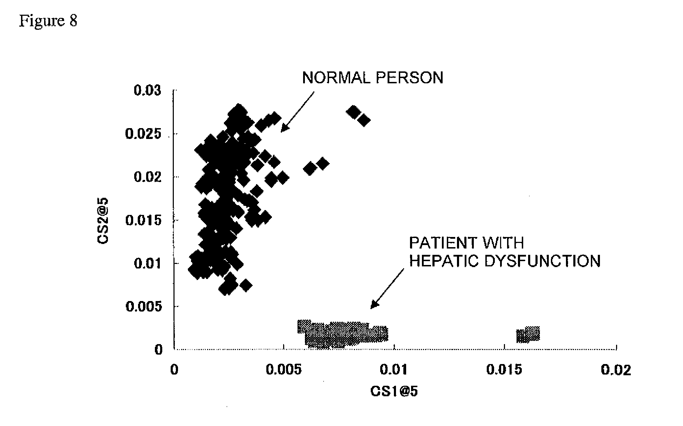

Fig. 8 shows a Coomans Plot obtained by the SIMCA analysis in the present Example. Table 14 as shown below shows a result of Interclass Distances, and Table 15 shows a result of Misclassification.

**[Table 14]**

| | CS1@5 | CS2@5 |
|---|---|---|
| CS1 | 0 | 5.046943 |
| CS2 | 5.046943 | 0 |

In Table 14, CS1 and CS2 show Class 1 and Class 2, respectively. "CS1@5" means that Class 1 uses 5 Factors (principal components).

**[Table 15]**

| | Pred1@5 | Pred2@5 | No match |
|---|---|---|---|
| Actual1 | 216 | 0 | 6 |
| Actual2 | 0 | 66 | 0 |

In Table 15, "Actual1" means that the actual class is "1", and hereinafter similarly. "Pred1" means that the class, which is anticipated using the analysis model prepared by SIMCA analysis in the present Example, is "1", and hereinafter similarly. "No match" is a numerical value of the case which is judged to be neither a patient with hepatic dysfunction nor a normal person. These results reveal that the analysis model prepared by SIMCA analysis can be used to judge rightly whether the subject is a patient with hepatic dysfunction or not.

Then, it was investigated whether the analysis model prepared by SIMCA analysis could be used to diagnose an unknown test sample which had been excluded from preparing the model or not. The results are shown in Table 16 below.

**[Table 16]**

| Excluded samples | | | |
|---|---|---|---|
| | PredCS1@5 | PredCS2@5 | No match |
| ActualCS1 | 45 | 0 | 0 |
| ActualCS2 | 0 | 35 | 10 |
| Unmodeled | 0 | 0 | 0 |

In Table 16, "ActualCS1" means that the actual class is "1", and hereinafter similarly. "PredCS1" means that the class, which is anticipated using the analysis model prepared by the SIMCA analysis, is "1", and hereinafter similarly. "No match" is a numerical value of the case which is judged to be neither a patient with hepatic dysfunction nor a normal person. "Excluded samples" is a result of unknown samples to anticipate. Table 16 shows that the unknown samples can be diagnosed about a patient with hepatic dysfunction.

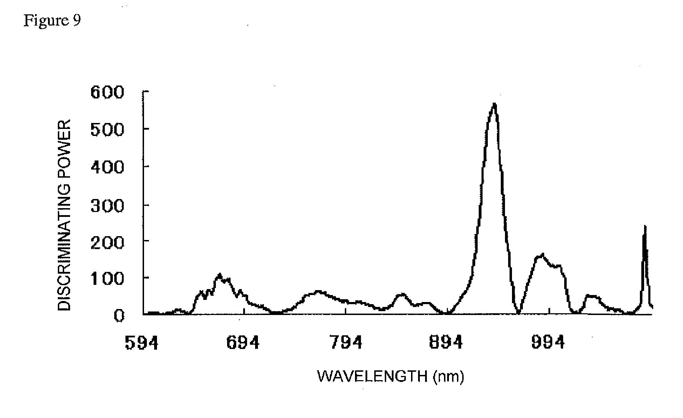

Fig. 9 shows the discriminating power (vertical axis) at wavelength (horizontal axis) which was obtained from the result by the SIMCA analysis. The figure shows that the higher discriminating power at a wavelength, the more different the wavelength between two classes. Thus, the wavelength, which corresponds to a sharp peak having a high discriminating power, is thought to be effective for discriminating serum between a normal person and a patient with hepatic dysfunction. Therefore, the identification with the wavelength taught by the SIMCA analysis allows simple, prompt, and accurate diagnosis of a patient with hepatic dysfunction.

The analysis model prepared in this way is stored as a file, which is called out for examining/diagnosing an unknown test sample to anticipate in which class it is classified. This allows simple and prompt examination/diagnosis of a patient with hepatic dysfunction.

From results in patients with anticipated diabetes, diabetic nephropathy, the other nephropathy than diabetic renal dysfunction, and hepatic dysfunction, the analysis model can be used to judge rightly each of them. In addition to the diseases as described above, hyperlipemia, heart disease and the like are also judged by the similar method, and their results can be totally judged to determine lifestyle related disease.

### Industrial Applicability

As described above, the present invention can examine/identify objectively, simply, promptly, and accurately lifestyle related disease, and thus can be used widely for examination/diagnosis of lifestyle related disease.

## Claims

1. A method for qualitative or quantitative examination/identification of lifestyle related disease, wherein the test sample from a human or other animal subject is irradiated with light having a wavelength of a region of 400 nm- 2500 nm or a part of the region, of which the reflection light, the transmission light, or the transmission reflection light is then detected to give spectroscopic data of absorbance, and afterward a previously prepared analysis model is used to analyze the absorbance at the whole or specific wavelength used for the measurement.

2. The method for examination/identification according to Claim 1, wherein one or two or more items of diabetes, renal dysfunction, hepatic dysfunction, hypertension, hyperlipemia, obesity, heart disease, and brain stroke are identified in association with diagnosis or progression of the lifestyle related disease.

3. The method for examination/identification according to Claim 1 or 2, wherein the test sample is a part of a living body including blood (such as blood plasma or blood serum), urine, the other body fluid, tissue, tissue extract, and ear, top ends of finger or toe.

4. The method for examination/identification according to any one of Claims 1 to 3, wherein the absorption spectroscopic data at two or more wavelengths, which are selected from a plurality of ± 5 nm wavelength regions of wavelengths selected from the group consisting of 817 nm, 921-959 nm, 987-1004 nm, 1008-1018 nm, 1028 nm, and 1040 nm, is used to analyze for examination/identification of the diabetes.

5. The method for examination/identification according to any one of Claims 1 to 3, wherein the absorption spectroscopic data at two or more wavelengths, which are selected from a plurality of ± 5 nm wavelength regions of wavelengths selected from the group consisting of 835 nm, 908-912 nm, 917-963 nm, 993-1002 nm, 1008 nm-1034 nm, 1040 nm, and 1060 nm, is used to analyze for examination/identification of the diabetic renal dysfunction.

6. The method for examination/identification according to any one of Claims 1 to 3, wherein the absorption spectroscopic data at two or more wavelengths, which are selected from a plurality of ± 5 nm wavelength regions of wavelengths selected from the group consisting of 914-915 nm, 919-967 nm, 994 nm, 1008 nm, 1012-1014 nm, 1018 nm, 1024 nm, 1030 nm, and 1034 nm, is used to analyze for examination/identification of the other nephropathy (nephrosclerosis, glomerular nephritis, IgA nephritis, and the like) than the diabetic renal function.

7. The method for examination/identification according to any one of Claims 1 to 3, wherein the absorption spectroscopic data at two or more wavelengths, which are selected from a plurality of ± 5 nm wavelength regions of wavelengths selected from the group consisting of 667-679 nm, 917-955 nm, 975-1005 nm, and 1083-1085 nm, is used to analyze for examination/identification of the hepatic dysfunction.

8. An analysis model for use in the method according to any one of Claims 1 to 7.

9. A program for examination/diagnosis of lifestyle related disease, wherein the program allows a computer to execute preparing, updating the analysis model for use in the method according to any one of Claims 1 to 7, or examining/diagnosing using the prepared analysis model.

10. An examination/diagnosis apparatus comprising:
an irradiator for irradiating the test sample from a human or other animal subject with light having a wavelength of a region of 400 nm to 2500 nm or a part of the region;
a spectroscope for spectroscoping before or after the irradiating and a detector for detecting the reflection light, the transmission light, or the transmission reflection light of the light irradiated on the test sample; and
a data analyzer for using a previously prepared analysis model to analyze the spectroscopic data of the absorbance(s) at a specific wavelength or over the whole wavelengths, which is(are) obtained by the detector, thereby to examine/diagnose qualitatively and quantitatively lifestyle related disease.

11. The examination/diagnosis apparatus according to Claim 10, wherein one or two or more items of diabetes, renal dysfunction, hepatic dysfunction, hypertension, hyperlipemia, obesity, heart disease, and brain stroke are identified in association with diagnosis or progression of the lifestyle related disease.

12. The apparatus for examination/ diagnosis according to Claim 10 or 11, wherein the test sample is a part of a living body including blood (such as blood plasma or blood serum), urine, the other body fluid, tissue, tissue extract solution, and a part of living body such as ear, top ends of finger or toe.

13. The apparatus for examination/diagnosis according to any one of Claims 10 to 12, wherein the absorption spectroscopic data at two or more wavelengths, which are selected from a plurality of ± 5 nm wavelength regions of wavelengths selected from the group consisting of 817 nm, 921-959 nm, 987-1004 nm, 1008-1018 nm, 1028 nm, and 1040 nm, is used to analyze for examination/identification of the diabetes.

14. The apparatus for examination/diagnosis according to any one of Claims 10 to 12, wherein the absorption spectroscopic data at two or more wavelengths, which are selected from a plurality of ± 5 nm wavelength regions of wavelengths selected from the group consisting of 835 nm, 908-912 nm, 917-963 nm, 993-1002 nm, 1008 nm-1034 nm, 1040 nm, and 1060 nm, is used to analyze for examination/identification of the diabetic renal dysfunction.

15. The apparatus for examination/diagnosis according to any one of Claims 10 to 12, wherein the absorption spectroscopic data at two or more wavelengths, which are selected from a plurality of ± 5 nm wavelength regions of wavelengths selected from the group consisting of 914-915 nm, 919-967 nm, 994 nm, 1008 nm, 1012-1014 nm, 1018 nm, 1024 nm, 1030 nm, and 1034 nm, is used to analyze for examination/identification of the other nephropathy (nephrosclerosis, glomerular nephritis, IgA nephritis, and the like) than the diabetic renal dysfunction.

16. The apparatus for examination/diagnosis according to any one of Claims 10 to 12, wherein the absorption spectroscopic data at two or more wavelengths, which are selected from a plurality of ± 5 nm wavelength regions of wavelengths selected from the group consisting of 667-679 nm, 917-955 nm, 975-1005 nm, and 1083-1085 nm, is used to analyze for examination/identification of the hepatic dysfunction.
